(19) Europäisches Patentamt
European Patent Office
Office européen des brevets

(11) Publication number: **0 199 474 A2**

(12) # EUROPEAN PATENT APPLICATION

(21) Application number: 86302349.5

(22) Date of filing: 27.03.86

(51) Int. Cl.[4]: C 07 D 249/08
A 01 N 43/653

(30) Priority: 22.04.85 GB 8510195

(43) Date of publication of application:
29.10.86 Bulletin 86/44

(84) Designated Contracting States:
AT BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Griffin, David Alan
12 Orion Roman Hill
Bracknell Berkshire(GB)

(72) Inventor: Mannion, Sally Kim
49 Wykeham Road Earley
Reading Berkshire(GB)

(74) Representative: Ricks, Michael James et al,
Imperial Chemical Industries PLC Legal Department:
Patents PO Box 6 Bessemer Road
Welwyn Garden City Herts, AL7 1HD(GB)

(54) **Substituted triazolyl butanoates as plant growth regulators.**

(57) Compounds of formula:

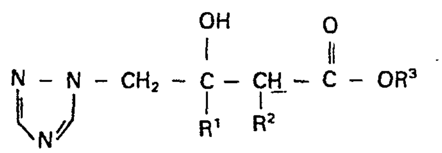

and stereoisomers thereof, wherein $R^1$ is halophenyl; $R^2$ is methyl and $R^3$ is $C_{1-3}$ alkyl; have fungicidal and plant growth regulating activity.

FIG. 1. THE EFFECT OF TEST CHEMICALS ON THE HEIGHT OF RICE (28 DAT)

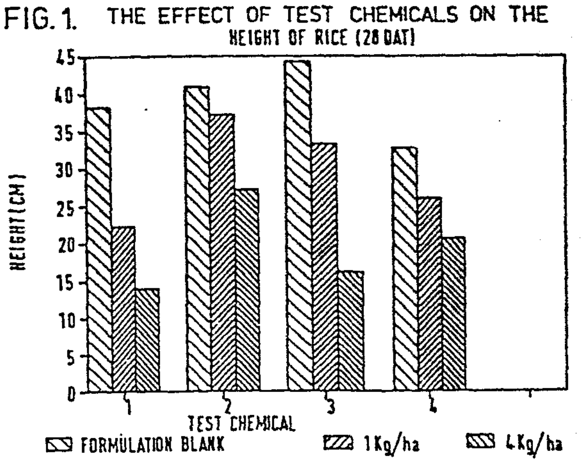


EP 0 199 474 A2

PP 33450/EP



JITLE MODIFIED
see front page

# HETEROCYCLIC COMPOUNDS

This invention relates to triazole compounds useful as plant growth regulators, to a process for preparing them, to plant growth regulating compositions containing them, and to methods of using them to regulate plant growth.

In USP 4394151 these are described compounds having the general formula (A) :

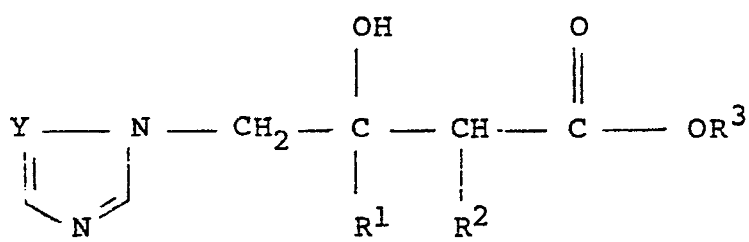

formula (A)

and stereoisomers thereof, wherein Y is -CH= or =N-, $R^1$ is alkyl, cycloalkyl or optionally substituted phenyl and $R^2$ and $R^3$, which may be the same or different, are hydrogen, alkyl, cycloalkyl, (e.g. cyclopropyl, cyclopentyl or cyclohexyl), optionally substituted phenyl or optionally substituted benzyl, or together form a lactone ring; and acid addition salts, ethers, esters and metal complexes thereof.

It has now been found that a certain narrow, novel, group of compounds embraced within the foregoing broad generic class of triazoles and imidazoles possesses substantially higher plant growth regulating activity than those disclosed in USP 4394151. This higher activity is also combined with superior symptomology, i.e. a lower level of undesirable effects upon plants.

According to the present invention we provide triazole derivatives having the general formula (I) :

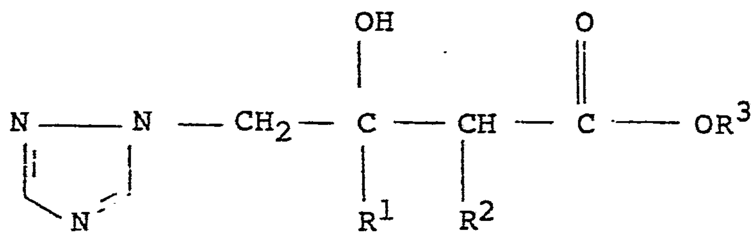

(I)

and stereoisomers thereof, wherein $R^1$ is halophenyl; $R^2$ is methyl; and $R^3$ is methyl, ethyl or propyl (iso or normal), and acid addition salts, ethers, esters and metal complexes thereof.

When $R^1$ is halophenyl it may be substituted with one two or three, halogen atoms eg. Cl,F,Br or I. Preferably these substituents are located at the 4-position or 2,4-positions. Chlorine is the preferred halogen. 2,4-Dichlorophenyl is preferred over 4-chlorophenyl.

The compounds of the invention can contain chiral centres. Such compounds are generally obtained in the form of racemic mixtures. However, these and other mixtures can be separated into the individual isomers by methods known in the art and this invention embraces such isomers.

Examples of the compounds of the invention are shown in Table 1. These conform to formula I.

TABLE 1

| COMPOUND NO | $R^1$ | $R^2$ | $R^3$ | MELTING POINT (°C) |
|---|---|---|---|---|
| 1 | 2,4-dichlorophenyl | $CH_3$ | ethyl | 99-101 |
| 2 | 2,4-dichlorophenyl | $CH_3$ | methyl | 114-118 |
| 3 | 2,4-dichlorophenyl | $CH_3$ | n-propyl | 92-95 |
| 4 | 2,4-dichlorophenyl | $CH_3$ | i-propyl | 95-98.4 |
| 5* | 2,4-dichlorophenyl | $CH_3$ | tertiary-butyl | Oil ($n_D^{20}$ 1.5298) |

* This compound is the closest, chemically, from the invention defined in USP 4394151 in respect of which this invention is one of selection.

NB. Sharp melting points were not obtainable in view of traces of the other diastereomer remaining even after repeated HPLC (chromatography).

The compounds of general formula (I) may be produced by reacting a compound of general formula (II) with (III) in a Reformatsky reaction using zinc dust or a zinc-copper couple.

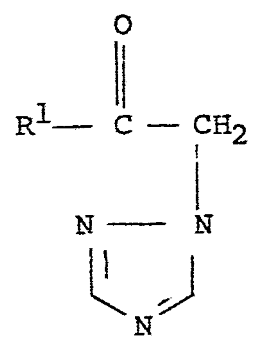

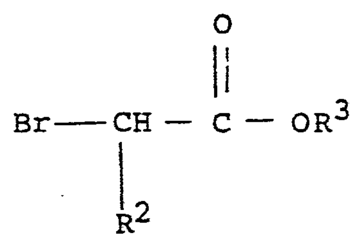

(II) (III)

They may also be made by reacting compounds of formula (II) with compounds of formula (IV) in the presence of a strong base such as lithium bis(trimethylsilyl)amide or lithium di-isopropylamide or lithium isopropyl-cyclohexylamide.

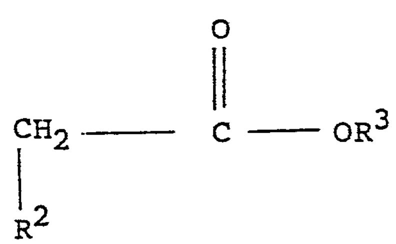

(IV)

The compounds of the invention may also be prepared by the process described in European Patent Application No. 8230635 (Patent Publication No. EP 86916).

The compounds of general formula (II) and (III) may be made by methods set out in the literature.

Suitably the compounds of general formula (II) and (III) are refluxed in a convenient solvent such as THF with zinc powder. The product can be isolated by

pouring the reaction mixture into water and recrystallising the solid formed from a convenient solvent.

The salts, ethers, esters and metal complexes of the compounds of general formula (I) can be prepared from the latter in known manner. For example, the complexes can be made by reacting the uncomplexed compound with a metal salt in a suitable solvent.

The compounds, and their derivatives as defined above, have plant growth regulating activities.

The plant growth regulating effects of the compounds are manifested as, for example, by a stunting or dwarfing effect on the vegetative growth of woody and herbaceous mono- and di-cotyledonous plants. Such stunting or dwarfing may be useful, for example, in peanuts, cereals such as wheat and barley, oil seed rape, field beans, sunflowers, potatoes and soya bean where reduction in stem height, with or without further advantageous effects such as stem strengthening, thickening and shortening, internode shortening, increased buttress root formation and more erect stem and leaf orientation, may reduce the risk of lodging and may also permit increased amounts of fertiliser to be applied. Compounds which are particularly useful in leaf orientation eg. making the leaves of wheat and barley plants more erect, are the compounds numbered 1, 2, 3, and 4 in Table I above. The stunting of woody species is useful in controlling the growth of undergrowth under power lines etc. Compounds which induce stunting or dwarfing may also be useful in modifying the stem growth of sugar cane thereby increasing the concentration of sugar in the cane at harvest; in sugar cane, the flowering and ripening may be controllable by applying the compounds. Stunting of peanuts can assist in harvesting. Growth retardation of grasses can help maintenance of grass swards. Examples of suitable grasses are Stenotaphrum secundatum (St. Augustine grass), Cyanosurus cristatus, Lolium multiflorum and perenne, Agrostis tenuis, Cynodon dactylon (Bermuda grass), Dactylis

*glomerata*, *Festuca* spp. (eg. *Festuca rubra*) and *Poa* spp. (eg. *Poa pratense*). The compounds may stunt grasses without significant phytotoxic effects and without deleteriously affecting the appearance (particularly the colour) of the grass; this makes such compounds attractive for use on ornamental lawns and on grass verges. They may also have an effect on flower head emergence in, for example, grasses. The compounds can also stunt weed species present in the grasses; examples of such weed species are sedges (eg. *Cyperus* spp.) and dicotyledonous weeds (eg. daisy, plantain, knotweed, speedwell, thistle, docks and ragwort). The growth of non-crop vegetation (eg. weeds or cover vegetation) can be retarded thus assisting in the maintenance of plantation and field crops. In fruit orchards subject to soil erosion, the presence of grass cover is important. However excessive grass growth requires substantial maintenance. The compounds of the invention could be useful in this situation as they could restrict growth without killing the plants which would lead to soil erosion; at the same time the degree of competition for nutrients and water by the grass would be reduced and this could result in an increased yield of fruit. In some cases, one grass species may be stunted more than another grass species; this selectivity could be useful, for example, for improving the quality of a sward by preferential suppression of the growth of undesirable species.

The dwarfing may also be useful in miniaturising ornamental, household, garden and nursery plants (eg. poinsettias, chrysanthemums, carnations, tulips and daffodils).

As indicated above, the compounds can also be used to stunt woody species. This property can be used to control hedgerows or to shape or reduce the need for pruning, of fruit trees (eg. apples, pears, cherries, peaches, vines etc). Some coniferous trees are not significantly stunted

by the compounds so the compounds could be useful in controlling undesirable vegetation in conifer nurseries.

The plant growth regulating effect may (as implied above) manifest itself in an increase in crop yield; or in an ability in orchards and other crops to increase fruit set, pod set and grain set.

In the potato, vine control in the field and inhibition of sprouting in the store may be possible.

Other plant growth regulating effects caused by the compounds include alteration of leaf angle and changes in leaf morphology (both of which may permit increased light interception and utilization) and promotion of tillering in monocotyledonous plants. Improved light interception is of value in all major world crops, eg. wheat, barley, rice, maize, soya, sugarbeet, potatoes, plantation crops and orchard crops. The leaf angle effect may be useful for example in altering the leaf orientation of, for example, potato crops thereby letting more light into the crops and inducing an increase in photosynthesis and tuber weight. By increasing tillering in monocotyledonous crops (eg. rice), the number of flowering shoots per unit area may be increased thereby increasing the overall grain yield of such crops. In addition better control and modification of hierarchical relationships is possible both in vegetative and reproductive stages of monocotyledonous and dicotyledenous plant growth, especially in cereals such as wheat, barley, rice and maize, whereby the number of flowering shoots per unit area may be increased and the size distribution of grains within the ear may be modified in such a way as to increase yield. In the treatment of rice plants, or rice crops the invention compounds can be applied, eg. as granules or a granular formulation, for example as slow release granules, to nursery boxes, paddy water and other like cultivation loci and media. In grass swards, especially amenity grass, an increase in tillering

could lead to a denser sward which may result in increased resilience in wear; and to increased yields and better quality of forage grass, eg. improved digestability and palatability.

The treatment of plants with the compounds can lead to the leaves developing a darker green colour. In dicotyledonous plants such as soyabean and cotton, there may be promotion of sideshooting.

The compounds may inhibit, or at least delay, the flowering of sugar beet (and thereby may increase sugar yield) or otherwise modify the flowering patterns in many other crops. They may also reduce the size of sugar beet without reducing significantly the sugar yield thereby enabling an increase in planting density to be made. Similarly in other root crops (eg. turnip, swede, mangold, parsnip, beetroot, yam and cassava) it may be possible to increase the planting density.

The compounds could be useful in restricting the vegetative growth of cotton thereby leading to an increase in cotton yield. Crop yields may also be increased by improvement of the harvest index (ie. the harvested yield as a proportion of the total dry matter produced) by altering dry matter partitioning. This applies to all the aforementioned root, pod, cereal, tree, plantation and orchard crops.

The compounds may be useful in rendering plants resistant to stress since the compounds can delay the emergence of plants grown from seed, shorten stem height and delay flowering; these properties could be useful in preventing frost damage in countries where there is significant snow cover in the winter since then the treated plants would remain below snow cover during the cold weather. Further the compounds may cause drought or cold resistance in certain plants.

When applied as seed treatments at low rates and compounds can have a growth stimulating effect on plants.

In carrying out the plant growth regulating method of the invention, the amount of compound to be applied to regulate the growth of plants will depend upon a number of factors, for example the particular compound selected for use, and the identity of the plant species whose growth is to be regulated. However, in general an application rate of 0.1 to 15, preferably 0.1 to 5, kg per hectare is used. With the use of biodegradable polymeric slow release granules rates of 1 to 10g per hectare are feasible; whilst electrodynamic spraying techniques may also deploy lower rates of application. However, on certain plants even application rates within these ranges may give undesired phytotoxic effects. Routine tests may be necessary to determine the best rate of application of a specific compound for any specific purpose for which it is suitable.

The compounds may be used as such for plant growth regulating purposes but are more conveniently formulated into compositions for such usage. The invention thus further provides a plant growth regulating composition comprising a compound of general formula (I) as hereinbefore defined, or a salt, metal complex, ether or ester thereof; and optionally, a carrier or diluent.

The invention also provides a method of regulating plant growth which comprises applying to a plant, to seed of a plant or to the locus of a plant or seed, a compound, or salt, metal complex, ether or ester thereof, as hereinbefore defined.

The compounds, salts, metal complexes, ethers and esters can be applied in a number of ways, for example they can be applied, formulated or unformulated, directly to the foliage of a plant, or they can be applied also to bushes and trees, to seeds or to other medium in which plants, bushes or trees are growing or are to be planted, or they can be sprayed on, dusted on or applied as a cream or paste formulation, or they can be applied as a vapour; or as slow release granules. Application can be to any part of the

plant, bush or tree, for example to the foliage, stems, branches or roots, or to soil surrounding the roots, or to the seed before it is planted; or to the soil generally, to paddy water or to hydroponic culture systems. The invention compounds may also be injected into plants or trees and they may also be sprayed onto vegetation using electrodynamic spraying techniques.

The term "plant" as used herein includes seedlings, bushes and trees.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and

suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (eg. 2-ethoxyethanol and 2-butoxyethanol).

The compositions to be used as sprays may also be in the form of aerosols wherein the formulation is held in a container under pressure in the presence of a propellant, eg. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a micro-encapsulated form. They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities.

The compounds can be used as mixtures with fertilisers (eg. nitrogen-, potassium- or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising the

compound of general formula (I) or a salt or metal complex thereof.

The compositions may also be in the form of liquid preparations for use as dips or sprays which are generally aqueous dispersions or emulsions containing the active ingredient in the presence of one or more surfactants eg. wetting agent(s), dispersing agent(s), emulsifying agent(s) or suspending agent(s); or which are spray formulations of the kind suitable for use in electrodynamic spraying techniques. The foregoing agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), and the concentrate is to be diluted with water before use. These concentrates often should be able to withstand storage for prolonged periods and after such storage be capable of dilution with water in order to form aqueous preparations which remain homogeneous for a sufficient time to enable them to be applied by conventional and electrodynamic spray equipment. The concentrates may conveniently contain up to 95%, suitably 10-85%, for example 25-60%, by weight of the active ingredient(s). These concentrates suitably contain organic acids (eg. alkaryl or aryl sulphonic acids such as xylenesulphonic acid or dodecyl benzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% to 10%, or 0.01% to 10%, by weight of active ingredient(s) may be used.

The compositions of this invention can comprise also other compound(s) having biological activity, eg. compounds having similar or complementary fungicidal or plant growth activity or compounds having plant growth regulating, herbicidal or insecticidal activity.

The other fungicidal compound can be, for example, one which is capable of combating ear diseases of cereals (eg. wheat) such as <u>Septoria</u>, <u>Gibberella</u> and <u>Helminthosporium</u> spp., seed and soil borne diseases and downy and powdery

mildews on grapes and powdery mildew and scab on apple etc. These mixtures of fungicides can have a broader spectrum of activity than the compound of general formula (I) alone; further the other fungicide can have a synergistic effect on the fungicidal activity of the compound of general formula (I). Examples of the other fungicidal compound are imazalil, benomyl, carbendazim, thiophanate-methyl, captafol, captan, sulphur, triforine, dodemorph, tridemorph, pyrazophos, furalaxyl, ethirimol, tecnazene, dimethirimol, bupirimate, chlorothalonil, vinclozolin, procymidone, iprodione, metalaxyl, forsetyl-aluminium, carboxin, oxycarboxin, fenarimol, nuarimol, fenfuram, methfuroxan, nitrotal-isopropyl, triadimefon, thiabendazole, etridiazole, triadimenol, biloxazol, dithianon, binapacryl, quinomethionate, guazatine, dodine, fentin acetate, fentin hydroxide, dinocap, folpet, dichlofluanid, ditalimphos, kitazin, cycloheximide, dichlobutrazol, a dithiocarbamate, a copper compound, a mercury compound, 1-(2-cyano-2-methoxyiminoacetyl)-3-ethyl urea, fenaponil, ofurace, propiconazole, etaconazole and fenpropemorph and fenpropidene.

The compounds of general formula (I) can be mixed with soil, peat or other rooting media for the protection of plants against seed-borne, soil-borne or foliar fungal diseases.

Suitable insecticides are Pirimor, Croneton, dimethoate, Metasystox and formothion.

The other plant growth regulating compound can be one which controls weeds or seedhead formation, improves the level or longevity of the plant growth regulating activity of the compounds of general formula (I), selectively controls the growth of the less desirable plants (eg. grasses) or causes the compound of general formula (I) to act faster or slower as a plant growth regulating agent. Some of these other agents will be herbicides.

Examples of suitable plant growth regulating

compounds, which can display synergy in admixture, or use, with the invention compounds are the gibberellins (eg. $GA_3$, $GA_4$ or $GA_7$), the auxins (eg. indoleacetic acid, indolebutyric acid, naphthoxyacetic acid or naphthylacetic acid), the cytokinins (eg. kinetin, diphenylurea, benzimidazole, benzyladenine or benzylaminopurine), phenoxyacetic acids (eg. 2,4-D or MCPA), substituted benzoic acids (eg. triiodobenzoic acid), morphactins (eg. chlorfluorecol), maleic hydrazide, glyphosate, glyphosine, long chain fatty alcohols and acids, dikegulac, fluoridamid, mefluidide, substituted quaternary ammonium and phosphonium compounds (eg. chlormequat* chlorphonium or mepiquat chloride*), ethephon, carbetamide, methyl-3,6-dichloroanisate, daminozide*, asulam, abscisic acid, isopyrimol, 1-(4-chlorophenyl)-4,6-dimethyl-2-oxo-1,2-dihydropyridine-3-carboxylic acid, hydroxybenzonitriles (eg. bromoxynil), difenzoquat*, benzoylprop-ethyl 3,6-dichloropicolinic acid, and tecnazene. Synergy will be most likely to occur with those of the foregoing which are quaternary ammonium compounds and with those marked with an asterisk. Also mixtures with fenpentezol, triapenthanol and flurpirimidol. Mixtures of the compounds of the present invention with chlormequat are especially suitable, and such mixtures may contain for example from 1 part by weight of triazole derivative per 1 part by weight chlormequat to 1 part by weight of triazole derivative per 4 parts by weight chlormequat.

The use of the compounds of general formula (I) in conjunction with gibberellins can be useful where it is desired to reduce the plant growth regulating effects of the compounds (eg. where they are to be used as fungicides). Where the compounds are being applied to the soil surrounding the plants or to the roots of the plant, the plant growth regulating effects of the compounds may possibly be reduced by using also certain types of phenoxybenzoic acids and their derivatives.

The following Examples illustrate the invention; the temperatures are given in degrees Centrigrade (°C).

EXAMPLE I

This Example illustrates the preparation of ethyl 2-methyl-3-hydroxy-3-(2,4-dichlorophenyl)-4-(1,2,4-triazole-1-yl)butanoate (Compound No 1 of Table 1).

Lithium bis (trimethylsilyl)amide (50 $cm^3$ of a IM solution in THF) was added to dry THF (50 $cm^3$) under argon at -78°, and a solution of ethylpropionate (5.74 $cm^3$) in dry THF (25 $cm^3$) was then added dropwise over 15 minutes at this temperature . When the addition was complete the mixture was stirred at -78° for 15 minutes. A solution of 2-(1,2,4-triazol-1-yl)-2',4'-dichloroacetophenone (12.81g) in dry THF was added dropwise over one hour at -78°. When the addition was complete the mixture was stirred at -78° for one hour, then water (200 $cm^3$) was added. The resulting mixture was extracted with dichloromethane (3 x 200 $cm^3$). The combined organic layers were washed once with hydrochloric acid (1M, 100 $cm^3$), then with water until neutral; and finally dried over anhydrous sodium sulphate. Removal of the solvent left a pale brown oil which was triturated with ether. The solid was removed by filtration and the filtrate subjected to flash dry-column chromatography on 100g of silica 60. The first 250 $cm^3$ eluted were discarded. The following 350 $cm^3$ eluted were collected and concentrated to give an oil. This oil was purified by HPLC on silica using as eluant ethylacetate/ 60-80 petroleum ether/methanol in the ratio 6:4:0.5. The first band to be eluted was collected and yielded on evaporation an oil. This oil was dissolved in ether, stirred with activated charcoal and filtered through Hyflo. Removal of the solvent left 1.4g of a colourless solid. M.pt. 99.0-101.0.

| Microanalysis : | C | H | N | |
|---|---|---|---|---|
| | 50.29 | 4.78 | 11.73 | Expected |
| | 50.29 | 4.89 | 11.58 | Found |

The title compound has the structure:

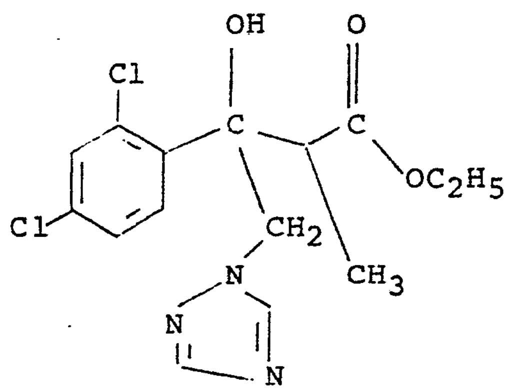

This compound is a single diastereomer (and is referred to as "isomer A"). The other diastereomer is contained in the second band to be eluted. It has a melting point of 95.0°-96.0° and is referred to as "isomer B".

The remaining compounds of Table I were similarly prepared. If desired or necessary the strong base, viz. lithium bis-(trimethylsilyl)-amide, may be replaced by similar bases eg. lithium di-isopropylamide or lithium isopropyl-cyclohexyl-amide.

EXAMPLE 2

The invention compounds (Nos 1 to 4 of Table I) have been compared with the closest representative of the prior art, USP 4394151. The structure of this closest representative (compound No 5 in Table I) is :-

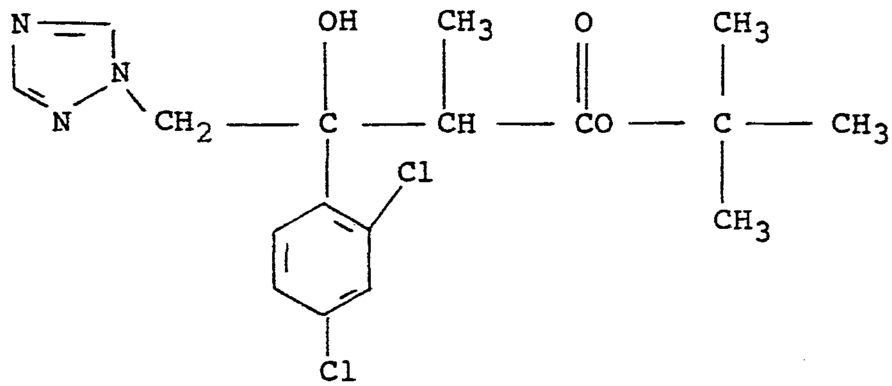

The compounds were tested on the plant growth regulator Whole Plant Screen (ie. using whole plants) and also on an intermediate stage test on rice and barley.

Methodology

1. Whole Plant Screen

All the compounds in Table I were tested on the Whole Plant Screen, however each compound was tested separately. All the results were scored visually by comparison with formulation blanks (ie. controls with no active ingredient) including results for a standard plant growth regulator which was also tested on each screen. By comparing the test compounds to the standard in each screen, and obtaining the value for percentage retardation compared to the standard, the relative activity of each compound was found. The standard chemical used was PP333 (paclobutrazol).

The species used on the screen and the growth stage at treatment are outlined in Table 2.

The compounds were applied at 4kg $ha^{-1}$ (ai) which is equivalent to 4000 ppm. The plants were scored visually for a range of plant growth regulation effects at 13 or 19 days after treatment depending on the time of year at which the plants were treated.

The test was kept under glasshouse conditions with supplementary lighting providing a 16 hour day length. The cereals were kept at 16°C/13°C, day/night temperatures while the remainder of the species were kept at 25°C/22°C, day/night temperatures.

II) Intermediate stage test on Rice and Barley

Compounds 1 to 4 from Table I were assessed for plant growth regulator effects on this screen. Compound 5 from Table I (the prior art compound) can be compared to compounds 1-4 since wheat and barley plants from the whole plant screen were visually assessed at the same number of days after treatment. The results are presented as the percentage reduction in height compared to the height of the formulation blank on that test. Thus the relative activity can be compared.

The compounds were applied at two rates 4kg $ha^{-1}$ (ai) and 1kg $ha^{-1}$ (ai) which is equivalent to 4000 ppm and 1000 ppm. The species used were spring barley cultivar ATEM and rice, cultivar ISHIKARI. The barley was treated at the 2-3 leaf stage and the rice was treated at 14 days after transplanting which is the 3-4 leaf stage.

Both species were assessed at 28 days after treatment for the height of the plant to the top leaf ligule on the mainstem.

The plants were kept under glass house conditions of 16 hours day length and 16°C/13°C day/night temperatures for barley, and the rice was kept in conditions of 14 hour day length and 25°C/23°C day/night temperatures.

<u>TABLE 2</u> <u>PLANT MATERIAL USED FOR WHOLE PLANT SCREEN</u>

| SPECIES | CODE | VARIETY | GROWTH STAGE AT TREATMENT | NO PLANTS PER 3" POT |
|---|---|---|---|---|
| Barley | BR | Atem | 1 - 1.5 leaves | 4 |
| Wheat | WW | Timmo | 1 - 1.5 leaves | 4 |
| Maize | MZ | Earliking | 4 leaves | 1 |
| Vines | VN | Chanez + unspecified | 4 leaves | 1 |
| Soya | SY | Amsoy | 1st trifoliate | 1 |
| Tomato | TO | Ailsa Craig | 1.5 - 2 leaves | 1 |
| Lettuce | LT | Verpia | 3 - 4 leaves | 1 |
| Sugar beet | SB | Amono | 2 leaves | 1 |
| <u>Agrostis tenius</u><br><u>Cynosurus cristatus</u><br><u>Dactylis glomerata</u> | AT<br>CC<br>DA | | cut to 2 cm 48 hours before treatment | Grown in rows in plastic punnets |

Results:

(i) Whole Plant Screen

In order to make a meaningful comparison across the screens in which the compounds were tested, the total retardation of the compound (i.e. the sum of all the individual retardation scores for each separate species tested) is expressed as a percentage of the total retardation of the standard compound on that test. The table of results demonstrate the superior level of activity of compounds 1,2,3 and 4 over compound 5, the prior art (USP 4394151) reference compound. The greatest amount of retardation was observed with compound 2 where R3 was methyl.

The total retardation observed for compounds 1-4 of Table I was significantly higher than the total retardation observed for the reference compound 5. Unacceptably high levels of apical damage were observed with compound 5 also. This is shown in the column marked "Apical Damage" in Table 3. Apical damage for each species was measured on a score of 0 - 3 where 3 represents total apical kill. The presence of the letter "A" after the test result in Table 3 indicates the observation of apical damage, although individual scores are not shown. The "Apical Damage" column shows the sum of the individual apical damages scores for each species. Thus with 11 species, the possible total score is 33 for each compound tested.

The claimed compounds are particularily useful for cereal retardation. This can be observed from Table 3 where there is a superior level of retardation on the monocotyledonous species with all the claimed compounds when compared to the reference compound 5.

TABLE 3 RESULTS OF THE WHOLE PLANT SCREEN ASSESSMENT

| TABLE I TEST COMPOUND NUMBER | SPECIES EFFECTS | | | | | | | | | | | APICAL DAMAGE | TOTAL RETARDATION | | |
|---|---|---|---|---|---|---|---|---|---|---|---|---|---|---|---|
| | WW | BR | MZ | AT | CC | DA | SY | SB | LT | TO | VN | TEST CPD. | TEST CPD. | STANDARD CPD. | % TEST/STD |
| 1 | 1T | | 1A | 1G | 1 | 1G | 2AG | 3A | 2AG | 3AG | 2AG | 10 | 17 | 23 | 73.9 |
| 2 | 1GT | 1G | 1 | 3 | 3 | 2 | 2GAT | 2GA | 2G | 3GA | 3GAT | 8 | 23 | 27 | 85.2 |
| 3 | 1 | 1 | 3GA | 1G | | 1 | 3GAT | 3GA | | 3GA | 3GA | 9 | 19 | 30 | 63.3 |
| 4 | 1G | 2 | 3G | 3G | 1G | 1G | 3GA | 3GA | 3GAT | 3GA | 3GA | 10 | 26 | 30 | 86.7 |
| 5 | G | | 2G | 1G | 1 | | 3A | 3GA | 2G | 3GA | 2A | 15 | 17 | 30 | 56.7 |

Key

Plant Height Reduction = 1 = 10-30%
= 2 = 31-60%
= 3 = 61-100%

G = Greening effect
T = Side shooting or tillering effect *

A = Apical kill.

This is scored on a 0-3 scale where 3 = total apical kill.
Column A refers to the amount of apical death out of a possible total score of 33.

(ii) Rice and Barley Intermediate Stage Test I

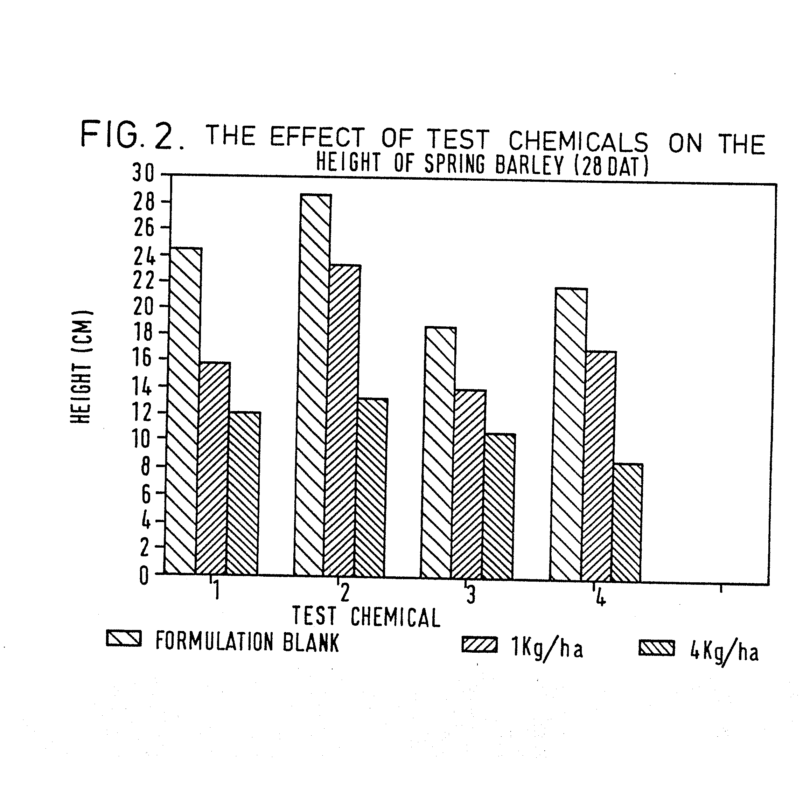

The results showing height to top leaf ligule on both rice and barley after treatment with compounds 1-5 are presented in Figures 1 and 2. Compound 5 was not tested in this series of experiments. However cereal plants from the Whole Plant Screen were visually assessed at 28 days after treatment and the result converted into the percentage retardation caused by the chemical when compared to the formulation blank. This was also done for compounds 1-4 and the results are presented in Table 4.

TABLE 4

% REDUCTION IN HEIGHT (COMPARED TO THE FORMULATION BLANK)

| | BARLEY | | RICE | | WHEAT |
|---|---|---|---|---|---|
| Compound No. | 1kg/ha | 4kg/ha | 1kg/ha | 4kg/ha | 4kg/ha |
| 1 | 35.3 | 51.0 | 41.7 | 63.7 | - |
| 2 | 18.3 | 54.0 | 9.3 | 33.6 | - |
| 3 | 24.9 | 43.0 | 25.0 | 64.2 | - |
| 4 | 22.2 | 61.0 | 20.7 | 33.8 | - |
| 5 | - | 8.3 | - | - | 0 |

The reference compound of USP 4394151 (compound No 5 of Table I) showed weak retardant activity on barley at 4kg/ha and was inactive on wheat at the same rate. Compounds 1-4 show greatly superior retardant activity even at the lower application rate of 1kg/ha on barley and rice. The spectrum of activity of the compounds was slightly different on each species. On barley, there was a peak of plant growth regulator activity when R3 signified a methyl, ethyl or isopropyl group; in contrast on rice the plant growth regulator activity was greatest when R3 was ethyl or propyl. The most active compound at the lower rate on both species was when R3 was ethyl i.e. for compound No 1 of Table I.

Discussion:

The compounds of the present invention were significantly more active as plant growth regulators than the reference compound No 5 of Table I in the Whole Plant Screen and barleyand rice intermediate test. From the morphological scores taken at 19 days after treatment in the Whole Plant Screen, compound No 5 of Table I caused a significantly greater amount of apical damage than was observed with the test compounds. The most active plant growth regulators at 19 days after treatment were compound 2 where $R^3$ is methyl and compound 4 where $R^3$ is iso-propyl. In the barley and rice intermediate test, on barley, the compounds where $R^3$ is methyl, ethyl and isopropyl were very active growth retardants. On rice there was equivalent retardant activity from the compounds where $R^3$ is methyl and isopropyl with the peak of retardant activity from the ethyl and n-propyl compounds, numbers 1 and 3.

The foregoing results are presented graphically in Figures 1 (rice plants) and 2 (spring barley plants). The vertical co-ordinate gives the height of the plant in centimetres 28 days after treatment whilst the horizontal co-ordinate gives the treatments for each of the four compounds tested. Compounds Nos 1, 2, 3 and 4 of Table I are presented from left to right, the first (ie. left-hand) column of each of the four sets of three columns being in each case a formulation blank (i.e. the formulation used without any active compound present). The second and third columns (from left to right) representing rates of application of test chemical of 1 kilogram per hectare and 4 kilograms per hectare.

The difference between the height of the plants sprayed with the formulation only and the height of the plants sprayed with the test chemical(s) is presented as a percentage reduction in height in Table 4 for each species.

The reference compound 5 is compared to compounds 1, 2, 3 and 4. The table shows clearly the superior retardant activity of compounds 1, 2, 3 and 4 over compound 5.

The compounds of the present invention are particularly useful as cereal (wheat, barley and rice) stem retardants. This makes them ideal for use as anti-lodging agents in crops where large yield losses occur from lodging every year.

EXAMPLE 3

This Example illustrates the use of mixtures of compounds of the present invention with chlormequat, a quaternary ammonium plant growth regulator.

Compound No 1 of Table 1 was tested on spring barley, both alone and in admixture with chlormequat. The spring barley cultivar Atem was grown in John Innes Potting compost in 3 inch pots with 4 plants per pot. The plants were treated at the 2-3 leaf stage and then placed in 16°C/13°C day/night temperatures and 16 hours day length for 20 days until assessment. A quantitative measurement of average height to the top-most leaf ligule was taken for each of eight replicates per treatment.

The following treatments were assessed :-

(a) compound no 1 applied at a rate of 4 kg ha $^{-1}$;
(b) compound no 1 applied at a rate of 2 kg ha $^{-1}$;
(c) chlormequat applied at a rate of 2 kg ha $^{-1}$; and
(d) a mixture of equal proportions of compound no 1 and chlormequat applied at an equivalent rate of 2 kg ha $^{-1}$ compound no 1 and 2 kg ha $^{-1}$ chlormequat.

A formulation blank containing no active ingredient was included in each test and the results are given in Table 5 expressed as the percentage reduction in height compared to plants treated with the formulation blank.

TABLE 5

| Treatment | Composition | Rate of application (kg ha $^{-1}$) | % reduction in height compared to formulation blank |
|---|---|---|---|
| (a) | Cpd 1 | 4 | 60.3 |
| (b) | Cpd 1 | 2 | 38.4 |
| (c) | chlormequat | 2 | 19.1 |
| (d) | chlormequat<br>Cpd 1 | 2<br>2 | 70.9 |

The results show that the mixture of chlormequat and compound No 1 was more active than would be expected on the basis of replacing half of compound no 1 in treatment (a) by an equal amount of the much less active chlormequat.

MJR/aji
PP 33450

1. A triazole derivative having the general formula (I) :

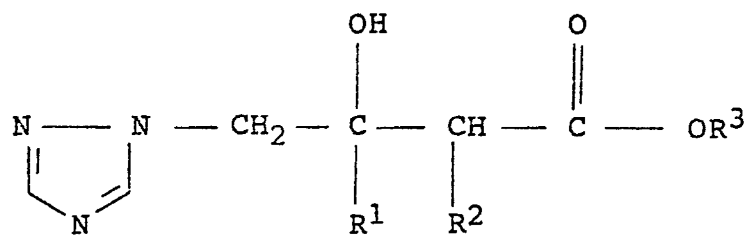

(I)

and stereoisomers thereof, wherein $R^1$ is halophenyl; $R^2$ is methyl; and $R^3$ is methyl, ethyl, isopropyl or normal propyl, and acid addition salts, ethers, esters and metal complexes thereof.

2. A triazole derivative according to claim 1 wherein the halophenyl is chlorophenyl.

3. A triazole derivative according to claim 1 or 2 wherein the halophenyl is substituted with one halogen atom located at the 4- position or two substituents located at the 2, 4- positions.

4. A triazole derivative according to claim 1 wherein $R^1$ is 2,4-dichlorophenyl.

5. A composition comprising a triazole derivative according to any of claims 1 to 4 in admixture with chlormequat.

6. A composition according to claim 5 wherein the composition comprises from 1 part by weight of triazole derivative per 1 part by weight of chlormequat to 1 part by weight of triazole derivative per 4 parts by weight of chlormequat.

7. A process for preparing compounds of general formula (I) as defined in claim 1 which comprises reacting a compound of general formula (II) with a compound of general formula (III) :

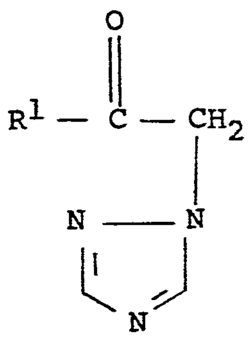

(II)

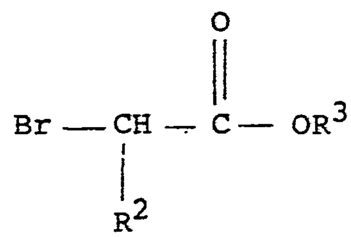

(III)

wherein $R^1$, $R^2$ and $R^3$ are as defined in claim 1, in a Reformatsky reaction using zinc dust or a zinc-copper couple.

8. A process according to claim 7 wherein the compounds of general formula (II) and (III) are refluxed in a solvent with zinc powder and the product is isolated by pouring the reaction mixture into water and recrystallising the solid formed from a solvent.

9. A plant growth regulating composition comprising, as an active ingredient, a compound as defined in any of claims 1 to 5 and a carrier or diluent for the active ingredient.

10. A method of regulating plant growth which comprises applying to the plant, to seed of the plant, or to the locus of the plant or seed, a compound as defined in any of claims 1 to 5 or a composition as defined in claim 6 or 9.

0199474

1 / 2

FIG. 1. THE EFFECT OF TEST CHEMICALS ON THE HEIGHT OF RICE (28 DAT)

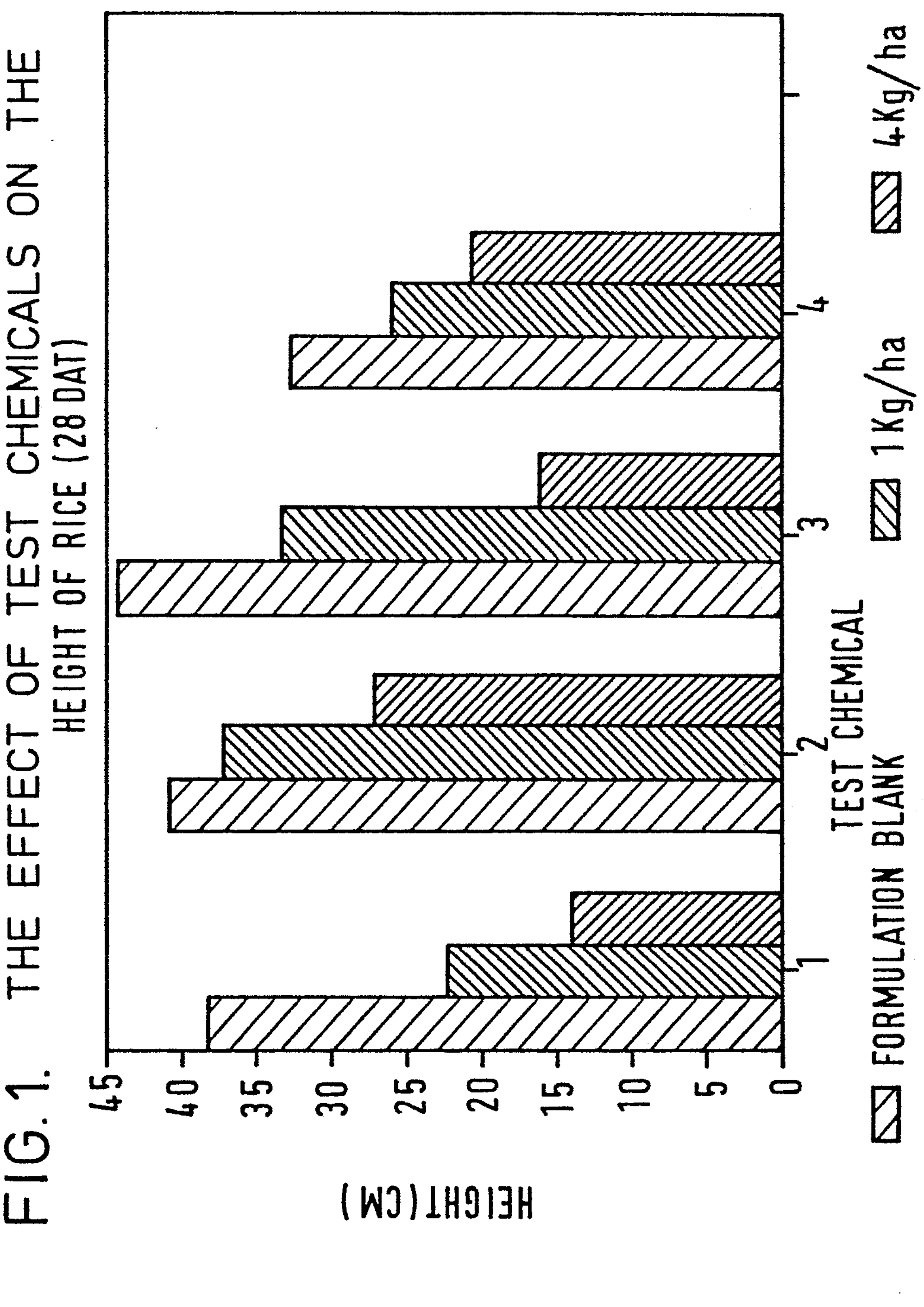

FIG. 2. THE EFFECT OF TEST CHEMICALS ON THE HEIGHT OF SPRING BARLEY (28 DAT)